# EUROPEAN PATENT APPLICATION

(11) **EP 2 615 148 A1**
(43) Date of publication of application: **17.07.2013**
(21) Application number: 11823546.4
(22) Date of filing: 06.09.2011
(51) Int. Cl.: C09K 3/00, A61L 9/00, A61L 9/01, B01D 53/86, B01J 35/02

(54) **ALLERGEN INACTIVATOR, ALLERGEN INACTIVATOR COMPOSITION, COATED MATERIAL, AIR PURIFICATION FILTER, AND ALLERGEN INACTIVATION METHOD**

(30) Priority: 08.09.2010 JP 2010201219
(71) Applicant: Panasonic Corporation, Osaka 571-8501 (JP); The University of Tokyo, Bunkyo-Ku Tokyo 113-8654 (JP)
(72) Inventor: MIKI, Shinichiro, Kadoma-shi Osaka 571-8686 (JP); KINUGAWA, Kensaku, Kadoma-shi Osaka 571-8686 (JP); HASHIMOTO, Kazuhito, Tokyo 113-8654 (JP); SUNADA, Kayano, Tokyo 113-8654 (JP)
(74) Representative: Müller-Boré & Partner Patentanwälte
(86) International application number: PCT/JP2011/070207
(87) International publication number: WO 2012/033071

(57) **Abstract**

The present invention provides an allergen inactivator that shows high allergen inactivating action. The allergen inactivator according to the present invention contains, as an active component, copper (I) oxide particulates having an average particle diameter of 10 µm or less. When an allergen comes into contact with the surfaces of the copper (I) oxide particulates, the allergen is degenerated to extinguish allergen activity.

## Description

### TECHNICAL FIELD

The present invention relates to an allergen inactivator, an allergen inactivator composition containing the allergen inactivator, a coated product subjected to treatment by the allergen inactivator composition, an air purification filter including the allergen inactivator, and an allergen inactivating method.

### BACKGROUND ART

In recent years, there is a problem about asthma and allergies caused by allergens included in excreta of mites, dead bodies of mites, cedar pollens, and the like. An allergen means protein (including glycoprotein) that idiosyncratically reacts mainly to a human antibody. According to the problem, people show more concern about the influence of a living environment on health. Measures against indoor allergens are a significant challenge for interior and building material manufactures and the like.

The indoor allergens are present in the surfaces of building materials such as fittings, floors and walls; furniture and bedclothes such as a sofa and a futon; stuffed toys; carpets; curtains; and the like. Therefore, there is a demand for a technique for suppressing occurrence of allergies by inactivating the allergens on the surfaces of these stuffs.

When a parson walks to fly up the allergens on the surfaces of the floor and the like, the allergens float in the air in the room. If the person inhales the allergens, an allergy such as asthma sometimes occurs. Therefore, an air purifiers or the like including a filter for removing allergens in the indoor air has been developed and placed in the market. However, there is a risk that the allergens captured by the filter scatter again from the filter or the allergens scatter during replacement of the filter and the allergens cause an allergy. Therefore, there is also a demand for a technique for effectively inactivating the allergens adhering to the filter of the air purifier or the like.

Conventionally, Patent document 1 has been provided a technique for inactivating allergens making use of catalyst particles having action for inactivating allergens.

Patent Document 1: Japanese Patent Application Laid-Open No. 2007-146453

However, a technique for inactivating allergen making use of a catalyst is still developing. There is a demand for an allergen inactivator that exhibits higher allergen inactivating action.

### DISCLOSURE OF THE INVENTION

The present invention has been made in view of the above problem and it is an object of the present invention to provide an allergen inactivator that exhibits high allergen inactivating action; an allergen inactivator composition that makes use of the allergen inactivator; a coated product that makes use of the allergen inactivator composition; an airpurification filter; andanallergen inactivating method.

An allergen inactivator according to the present invention contains, as an active component, copper (I) oxide particulates having an average particle diameter of 10 µm or less.

The allergen inactivator according to the present invention may further contains a photocatalyst material. The copper (I) oxide particulates are complexed with the photocatalyst material.

In the present invention, conduction band lower end potential of the photocatalyst material may be 0.16 V (vs. SHE, pH=0) or less.

An allergen inactivator composition according to the present invention may comprise: the allergen inactivator and a binder component.

In the present invention, amass ratio of the binder component with respect to a solid content amount of the allergen inactivator composition may be 95% or less.

A coated product according to the present invention comprises: an object to be treated and a coating film that covers the object. The coating film is formed of the allergen inactivator composition.

An air purification filter according to the present invention comprises: the allergen inactivator and a filter that carries the allergen inactivator.

An allergen inactivating method according to the present invention comprises steps of: preparing an allergen inactivator containing copper (I) oxide particulates as an active component; and bringing gas or liquid containing an allergen into contact with the surfaces of the copper (I) oxide particulates to degenerate the allergen and extinguish allergen activity.

In the allergen inactivating method according to the present invention, the allergen inactivator may further contain a photocatalyst material. The copper (I) oxide may be complexed with the photocatalyst material.

In the allergen inactivating method according to the present invention, conduction band lower end potential of the photocatalyst material may be 0.16 V (vs. SHE, pH=0) or less.

The allergen inactivating method according to the present invention may further comprise steps of: depositing the allergen inactivator on a surface of an object to be treated; and bringing the gas or the liquid containing the allergen into contact with the surfaces of the copper (I) oxide particulates on the surface of the object.

The allergen inactivating method according to the present invention may further comprise steps of: mixing the allergen inactivator with a binder component to prepare an allergen inactivator composition; forming a coating film made from the allergen inactivator composition on a surface of an object to be treated; and bringing the gas or the liquid containing the allergen into contact with the surfaces of the copper (I) oxide particulates exposed to a surface of the coating film.

In the allergen inactivatingmethod according to the present invention, a mass ratio of the binder component to a solid content amount of the allergen inactivator composition may be 95% or less.

The allergen inactivating method according to the present invention may further comprise steps of: preparing a filter as the object; causing the filter to carry the allergen inactivator; and causing air to pass through the filter to extinguish allergen activity in the air.

According to the present invention, it is possible to obtain an allergen inactivator that can effectively inactivate allergen.

It is possible to obtain an allergen inactivator composition containing the allergen inactivator and a binder.

It is possible to exhibit high allergen inactivating action on the surface of a coated product including a coating film formed of the allergen inactivator composition.

Further, it is possible to obtain an air purification filter by causing a filter to carry the allergen inactivator, and it is possible to effectively inactivate an allergen (s) in the air using the air purification filter.

With the allergen inactivating method according to the present invention, it is possible to effectively inactivate an allergen(s) in gas or liquid.

### BEST MODE FOR CARRYING OUT THE INVENTION

An allergen inactivator according to an embodiment contains copper (I) oxide particulates as an active component. If the allergen inactivator is used, an allergen comes into contact with surfaces of the copper (I) oxide particulates, and the allergen is degenerated. Therefore, allergen activity is extinguished. That is, the copper (I) oxide is coordinated to protein constituting the allergen to change the structure of the protein. Thereby the copper (I) oxide extinguishes a function of the protein specifically reacting to an antibody of a human. Therefore, the copper (I) oxide exhibits extremely excellent allergen inactivating action.

Irrespective of whether the copper (I) oxide has a crystal structure or is amorphous and, if the copper (I) oxide has the crystal structure, irrespective of the crystal structure, the copper (I) oxide shows high allergen inactivating action. Therefore, the crystal structure and the like of the copper (I) oxide are not particularly limited.

An average particle diameter of the copper (I) oxide particulates is desirably 10 µm or less, and more desirably 1 µm or less. The average particle diameter is a value measured by dynamic light scattering. An allergen inactivating function expresses on the surfaces of the copper (I) oxide particulates. Therefore, as the average particle diameter of the copper (I) oxide particulates decreases and a specific surface area thereof increases, the inactivating action of the allergen inactivator increases. Although a lower limit value of the average particle diameter of the copper (I) oxide particulates is not particularly limited, usually, the lower limit value is about 10 nm.

The allergen inactivator may contain a photocatalyst material together with the copper (I) oxide particulates.

The copper (I) oxide particulates are desirably complexed with the photocatalyst material. The complexing in this context means forming a state that a path for translocating electrons into the copper (I) oxide particulates is present, in which the electrons are generated by exciting the photocatalyst material with excitation light. A method of the complexing is not particularly limited. Examples of the method of the complexing include a method of kneading the copper (I) oxide particles and particles of the photocatalyst material with a mortar, a method of heating together with agitating the copper (I) oxide particles and the particles of the photocatalyst material in a solvent such as water, a method of precipitating the copper (I) oxide particulates on the surfaces of the particles of the photocatalyst material making use of a chemical reaction, and a method of reducing copper (II) oxide to the copper (I) oxide through reduction treatment after the photocatalyst material and the copper (II) oxide are complexed by the method explained above.

The copper (I) oxide has a characteristic that, when the copper (I) oxide is left untouched in the air for a long time, the copper (I) oxide is gradually oxidized to change to the copper (II) oxide. An allergen inactivating action of the copper (II) oxide is extremely weak compared with the copper (I) oxide. Therefore, when the copper (I) oxide is oxidized, a high allergen inactivating action may be lost. However, when the copper (I) oxide particulates are complexed with the photocatalyst material, even if the copper (I) oxide is oxidized to change to the copper (II) oxide, the copper (II) oxide is reduced to the copper (I) oxide because the electrons from the photocatalyst material excited by the excitation light are inj ected into the copper (II) oxide. Therefore, since the copper (I) oxide particulates are complexed with the photocatalyst material, the allergen inactivator according to this embodiment develops the high allergen inactivating action for a long period even in the air. Further, since an allergen is inactivated as well by oxidative decomposition action that develops when the excitation light is irradiated on the photocatalyst material, the action of the allergen inactivator increases.

In general, the photocatalyst material is particulates. Therefore, when the copper (I) oxide particulates are complexed with the photocatalyst material in an allergen inactivator composition containing the allergen inactivator and a binder component, a coating film formed of the allergen inactivator composition tends to become porous. When the coating film becomes porous in this way, the copper (I) oxide particulates included in the coating film more easily come into contact with the allergen. Therefore, when the copper (I) oxide particulates are complexed with the photocatalyst material, even if the content of the copper (I) oxide particulates is reduced, it is possible to exhibit the excellent allergen inactivating action in the coating film.

The photocatalyst is not particularly limited as long as the photocatalyst material in which, when light having energy larger than an energy gap between a conduction band and a valence electron band is irradiated, electrons in the valence electron band are excited and conduction electrons and holes can be generated. Specific examples of the photocatalyst material include oxides such as titanium oxide, tungsten oxide, zinc oxide, tin oxide, zirconium oxide, chrome oxide, molybdenum oxide, ruthenium oxide, germanium oxide, lead oxide, cadmium oxide, copper oxide, vanadium oxide, niobium oxide, tantalum oxide, manganese oxide, cobalt oxide, rhodium oxide, nickel oxide, rhenium oxide, and strontium oxide; oxides of these plurality of kinds of metals; and metal oxides doped with nitrogen and metal ions. The specific examples of the photocatalyst material are also cited as metal oxides which are carried photosensitization pigments and co-catalysts such as metal and metal salt on the surfaces of the metal oxides.

Conduction band lower end potential of the photocatalyst in the allergen inactivator is desirably 0.16 V (vs. SHE, pH=0) or less. 0.16V (vs. SHE, pH=0) is equal to the oxidation-reduction potential between copper monovalent ions and copper divalent ions. When the conduction band lower end potential of the photocatalyst is equal to or lower than the oxidation-reduction potential between the copper monovalent ions and copper divalent ions, electrons excited in a conduction band of photocatalyst have high reduction power enough for reducing the copper divalent ions, the copper (II) oxide in the allergen inactivator is easily reduced to change to the copper (I) oxide.

The valence electron band potential of the photocatalyst material is desirably 3 V (vs. SHE, pH=0) or more. The valence electron band potential of the photocatalyst material affects the strength of oxidizing power obtained when the photocatalyst is excited. The oxidizing power is stronger as the valence electron band potential is higher. In particular, when the valence electron band potential is 3 V (vs. SHE, pH=0) or more, the allergen is effectively oxidatively decomposed and the action of the allergen inactivator increases. However, when the valence electron potential is high and the conduction band lower end potential is low, the band gap increases and energy necessary for the exciting the photocatalyst increases. In this case, since the photocatalyst is excited by a shorter wavelength of light, environmental conditions for using the allergen inactivator would be limited.

Considering the above comprehensively, titanium oxide is optimum as the photocatalyst material.

When the allergen inactivator is deposited on the surface of an appropriate object to be treated, and when gas or liquid containing an allergen comes into contact with the surfaces of the copper (I) oxide particulates on the surface of the treated object by the allergen inactivator, the allergen activation in the gas or the liquid is effectively degenerated. Therefore, the allergen is inactivated.

When the allergen inactivator is, for example, sprayed on a filter having air permeability or inserted in the filter to be carried on the filter, an air purification filter that shows high allergen inactivating action is obtained.

An allergen inactivator composition is also obtained from the allergen inactivator. The allergen inactivator in the allergen inactivator composition may or may not contain the photocatalyst. The allergen inactivator composition desirably contains the allergen inactivator and further contains a binder component. The binder component is a component that is dried or hardened to fix the allergen inactivator.

The binder component is not particularly limited as long as the binder component is a usual compound for forming a harden film by an appropriate process such as drying, heating, ultraviolet ray irradiation, or electron beam irradiation to form a film.

When the allergen inactivator contains the photocatalyst, the binder component desirably includes a component having siloxane bond or a component that forms the siloxane bond through a reaction. Examples of the component having the siloxane bond include acrylic silicon resin, a silicone composition, and a partial hydrolysis polycondensation product of the acrylic silicon resin and the silicone composition. In this case, since the siloxane bond is less easily decomposed by the photocatalyst action, the durability of a coating film formed of the allergen inactivator composition is improved.

An amount of the binder component in the allergen inactivator composition is desirably 95% or less in a mass ratio based on a solid content amount of the allergen inactivator composition. The solid content amount of the allergen inactivator composition means mass after hardening and drying of the allergen inactivator composition. When the amount of the binder component is larger than 95%, most of the allergen inactivator in the allergen inactivator composition is covered by the binder component. Therefore, it is likely that the allergen less easily comes into contact with the allergen inactivator and the allergen inactivating action of the coating film formed of the allergen inactivator composition is not sufficiently exhibited.

In the allergen inactivator composition, when the copper (I) oxide particulates are complexed with the photocatalyst material, the coating film formed of the allergen inactivator composition tends to become porous as explained above. When the coating film becomes porous, the copper (I) oxide particulates included in the coating film more easily come into contact with the allergen. Therefore, when the copper (I) oxide particulates are complexed with the photocatalyst material, even if the content of the copper (I) oxide particulates is reduced, it is possible to exhibit the high allergen inactivating action in the coating film.

In the allergen inactivator composition, when the copper (I) oxide particulates are complexed with the photocatalyst material, a ratio of the copper (I) oxide particulates in the allergen inactivator composition is desirably 0.5% or more in a mass ratio based on the entire solid content amount of the allergen inactivator composition. By setting the ratio of the copper (I) oxide particulates to the mass ratio equal to or higher than 0.5%, a function of inactivating the allergen can be suitably obtained. The copper (I) oxide particulates assume a reddish brown color and have high opacifying power. Therefore, when the amount of the copper (I) oxide particulates exceeds 5% in the mass ratio based on the entire solid content amount of the allergen inactivator composition, a formed coating film is clearly colored in reddish brown. Therefore, when the coating film is required to be transparent, the complexing with the photocatalyst material is particularly useful because the complexing is effective for reducing the content of the copper (I) oxide particulates.

A lower limit value of the amount of the binder component in the allergen inactivator composition is not particularly limited. A coating property required of the coating film is determined according to, for example, an object to be subjected for coating treatment and an environment in which the object is used. A mass ratio of the binder component in the allergen activator composition is freely selected according to the coating property required of the coating film.

The allergen inactivator composition may contain an appropriate solvent in order to satisfy preferable coating properties according to necessity.

The allergen activator composition may contain a leveling agent, an antifoaming agent, a wetting agent, various pigments, and the like for the purpose of satisfying properties, such as an external appearance and coating aptitude, required of the allergen inactivator composition.

The coating treatment by the allergen inactivator composition is applied to an appropriate object to be treated, whereby a coated product is obtained. In the coating treatment applied to the obj ect to be treated, for example, after the allergen inactivator composition is applied to or impregnated in the surface of the object to be treated according to a type of the object to be treated, the allergen inactivator composition is hardened to form a film by an appropriate method of, for example, heating the allergen inactivator composition according to a condition such as a composition of the binder component and presence or absence of a solvent, whereby a coating film is formed.

The object to be treated is not particularly limited. However, examples of the object to be treated include furniture and bedclothes such as a sofa and a futon, stuffed toys, building materials such as a floor material, fiber and fiber products such as wall paper, a filter, a carpet, and a curtain.

When gas or liquid containing an allergen comes into contact with the surfaces of the copper (I) oxide particulates exposed on the surface of the coating film, the allergen in the gas or the liquid is effectively degenerated. Therefore, the allergen is inactivated. Therefore, excellent allergen inactivating action develops on the surface of the coated product. Consequently, the coated product is particularly effective for inactivating allergen which is included in so-called inhaled allergens such as indoor dust, scurf, pollens, fungi, and insects.

The present invention is not limited to the embodiment and can be variously modified without departing from the spirit of the invention.

### Examples

Specific examples of the present invention are explained. The present invention is not limited to the examples.

### Example 1

0.7 parts by mass of rutile-type titanium oxide (MT-150A manufactured by Tayca Corporation) and 1.4 parts by mass of copper (I) oxide (manufactured by Wako Pure Chemical Industries, Ltd., having an average particle diameter of 1.5 µm) were added to 17.9 parts by mass of deionized water and suspended to obtain a suspension. The suspension was heated to 90°C while being agitated by a stirrer and retained for one hour to obtain liquid containing an allergen inactivator in which the copper (I) oxide and the titanium oxide are complexed.

On the other hand, 5 parts by mass of tetraethoxysilane (manufactured by Wako Pure Chemical Industries, Ltd.), 0.8 parts by mass of deionized water, 0.07 parts by mass of HNO₃ water solution having concentration of 0.1 mol/L, and 74.13 parts by mass of ethanol were mixed in a reaction vessel and agitated for sixteen hours to obtain liquid containing a partial hydrolysis polycondensation product of tetraethoxysilane.

20 parts by mass of the liquid containing the allergen inactivator and 80 parts by mass of the liquid containing the partial hydrolysis polycondensation product of tetraethoxysilane were mixed and agitated for one hour to obtain an allergen inactivator composition.

The allergen inactivator composition was applied on a 50mm-squire clean glass plate by spin coating to form a coating film. The coating film was heated for thirty minutes at 100°C to be dried and hardened to obtain a coated product, which is a sample for evaluation.

### Example 2

1.4 parts by mass of copper (I) oxide (manufactured by Wako Pure Chemical Industries, Ltd., having an average particle diameter of 1.5 µm) was added to 18.6 parts by mass of deionized water and suspended to obtain liquid containing an allergen inactivator.

On the other hand, 5 parts by mass of tetraethoxysilane (manufactured by Wako Pure Chemical Industries, Ltd.), 0.7 parts by mass of rutile-type titanium oxide (MT-150A manufactured by Tayca Corporation), 0.8 parts by mass of deionized water, 0.07 parts by mass of HNO₃ water solution having concentration of 0.1 mol/L, and 73.43 parts by mass of ethanol were mixed in a reaction vessel and agitated for sixteen hours to obtain liquid containing a partial hydrolysis polycondensation product of tetraethoxysilane including rutile-type titanium oxide particulates.

20 parts by mass of the liquid containing the allergen inactivator and 80 parts by mass of the liquid containing the partial hydrolysis polycondensation product of tetraethoxysilane including the rutile-type titanium oxide particulates were mixed and agitated for one hour to obtain an allergen inactivator composition.

Thereafter, a coated product was obtained in the same manner as the example 1.

In this example, the titanium oxide is added to a reaction system during hydrolysis polycondensation reaction of tetraethoxysilane, whereby a thin film of silica is formed on the surface of the titanium oxide. Therefore, the copper (I) oxide and the titanium oxide are not complexed.

### Example 3

0.7 parts by mass of tin oxide (MT-150A manufactured by Wako Pure Chemical Industries, Ltd.) and 1.4 parts by mass of copper (I) oxide (manufactured by Wako Pure Chemical Industries, Ltd., having an average particle diameter of 1.5 µm) were added to 17.9 parts by mass of deionized water and suspended to obtain a suspension. The suspension was heated to 90°C while being agitated by a stirrer and retained for one hour to obtain liquid containing an allergen inactivator in which the copper (I) oxide and the tin oxide (IV) are complexed.

Thereafter, a coated product was obtained in the same manner as the example 1.

The conduction band lower end potential of the tin oxide is higher than 0.16 V (vs. SHE, pH=0).

### Example 4

1.4 parts by mass of copper (I) oxide (manufactured by Wako Pure Chemical Industries, Ltd., having an average particle diameter of 1.5 µm) was added to 18.6 parts by mass of deionized water and suspended to obtain an allergen inactivator.

Thereafter, a coated product was obtained in the same manner as the example 1.

### Example 5

0.15 parts by mass of copper (I) oxide (manufactured by Wako Pure Chemical Industries, Ltd., having an average particle diameter of 1.5 µm) was added to 19.85 parts by mass of deionized water and suspended to obtain an allergen inactivator.

Thereafter, a coated product was obtained in the same manner as the example 1.

### Example 6

0.06 parts by mass of copper (I) oxide (manufactured by Wako Pure Chemical Industries, Ltd., having an average particle diameter of 1.5 µm) was added to 19.94 parts by mass of deionized water and suspended to obtain an allergen inactivator.

Thereafter, a coated product for evaluation was obtained in the same manner as the example 1.

### Example 7

After 0.1 g of copper (I) oxide (manufactured by Wako Pure Chemical Industries, Ltd., having an average particle diameter of 1.5 µm) was sprayed on a commercially available glass filter (type G2: having a pore diameter of 40 to 50 µm and a filter diameter of 30 mm), the glass filter was cleaned three times by deionized water to filtrate fine copper (I) oxide particles. Further, the glass filter was dried for one hour at 60°C. Consequently, a glass filter carrying the copper (I) oxide particles was obtained. This glass filter was used as a sample for evaluation.

### Comparative example 1

5 parts by mass of tetraethoxysilane (manufactured by Wako Pure Chemical Industries, Ltd.), 0.8 parts by mass of deionized water, 0. 07 parts by mass of HNO₃ water solution having concentration of 0.1 mol/L, and 94.13 parts by mass of ethanol were mixed in a reaction vessel and agitated for sixteen hours to obtain liquid containing a partial hydrolysis polycondensation product of tetraethoxysilane.

The liquid containing the partial hydrolysis polycondensation product of tetraethoxysilane was applied on a 50mm-squire clean glass plate by spin coating. A coating film was heated for thirty minutes at 100°C to be dried and hardened to obtain a coated product, which is a sample for evaluation.

### Comparative example 2

5 parts by mass of tetraethoxysilane (manufactured by Wako Pure Chemical Industries, Ltd.), 1.4 parts by mass of rutile-type titanium dioxide (MT-150A manufactured by Tayca Corporation), 0.8 parts by mass of deionized water, 0.07 parts by mass of HNO₃ water solution having concentration of 0.1 mol/L, and 92. 73 parts by mass of ethanol were mixed in a reaction vessel and agitated for sixteen hours to obtain liquid containing a partial hydrolysis polycondensation product of tetraethoxysilane including rutile-type titanium dioxide particulates.

The liquid containing the partial hydrolysis polycondensation product of tetraethoxysilane including the rutile-type titanium dioxide particulates was applied on a 50mm-squire clean glass plate by spin coating. A coating film was heated for thirty minutes at 100°C to be dried and hardened to obtain a coated product, which is a sample for evaluation.

### Comparative example 3

1.4 parts by mass of copper (II) oxide (manufactured by Wako Pure Chemical Industries, Ltd.) was added to 18.6 parts by mass of deionized water and suspended to obtain a copper (II) oxide suspension.

5 parts by mass of tetraethoxysilane (manufactured by Wako Pure Chemical Industries, Ltd.), 0.8 parts by mass of deionized water, 0. 07 parts by mass of HNO₃ water solution having concentration of 0.1 mol/L, and 74.13 parts by mass of ethanol were mixed in a reaction vessel and agitated for sixteen hours to obtain liquid containing a partial hydrolysis polycondensation product of tetraethoxysilane.

20 parts by mass of the copper (II) oxide suspension and 80 parts by mass of the liquid containing the partial hydrolysis polycondensation product of tetraethoxysilane were mixed and agitated for one hour to obtain an allergen inactivator composition including the copper (II) oxide.

The allergen inactivator composition including the copper (II) oxide was applied on a 50mm-squire clean glass plate by spin coating. A coating film was heated for thirty minutes at 100°C to be dried and hardened to obtain a coated product including the copper (II) oxide, which is a sample for evaluation.

### Comparative example 4

A commercially available glass filter (type G2: having a pore diameter of 40 to 50 µm and a filter diameter of 30 mm) was directly used as a sample for evaluation.

### Performance evaluation

Performance evaluation explained below was carried out concerning the samples for evaluation obtained in the examples 1 to 6 and the comparative examples 1 to 3 in which the object to be treated was the glass plate.

### Pretreatment

### (a) Pretreatment condition 1

After a sample for evaluation was enclosed in a Tedlar bag of a bag size 3L together with an appropriate amount of pure air, an ultraviolet ray was irradiated on the sample for evaluation from a black light (Handy UV Lamp LUV-16 manufactured by AS ONE Corporation) for twenty-four hours to have ultraviolet ray radiation illuminance of 1 mW/cm² on the surface of the sample for evaluation.

### (b) Pretreatment condition 2

The sample for evaluation subjected to treatment under the pretreatment condition 1 was left untouched for twenty-eight days in a thermostatic chamber adjusted to temperature and humidity conditions of 30°C and 90% RH. Subsequently, an ultraviolet ray was irradiated on the sample for evaluation from the black light for twenty-four hours to have ultraviolet ray radiation illuminance of 1 mW/cm² on the surface of the sample for evaluation. The pretreatment condition 2 is processing for evaluating influence of oxidative degradation due to long-time exposure to the air.

### Allergen inactivation test

### (a) Test condition 1

An allergen (refined dermatophagoides allergen Derf 1 manufactured by ASAHI BREWERIES, LTD.) was added to a buffer solution (a solution obtained by diluting a biochemical buffer solution 20X PBS Tween-20 Buffer manufactured by Takara Bio Inc. by a factor of 20 with hyperpure water) to prepare allergen liquid having concentration of 33.3 ng/L.

After 0.4 mL of the allergen liquid was dripped on the sample for evaluation subjected to the pretreatment, the sample for evaluation was covered with a 40mm-square film.

Subsequently, an ultraviolet ray was irradiated on the sample for evaluation from the black light for twenty-four hours to have ultraviolet ray radiation illuminance of 0.1 mW/cm² on the surface of the sample for evaluation.

Subsequently, the allergen liquid on the sample for evaluation was collected. Allergen concentration in the allergen liquid was determined by an enzyme-linked immunosorbent assay method (ELISA method), an allergen concentration change in the allergen liquid was calculated in percentage, and a degree of inactivation of the allergen was evaluated.

### (b) Test condition 2

Under the test condition 1, the ultraviolet ray irradiation from the black light was not performed. Instead, the sample for evaluation was left untouched for twenty-four hours in a dark place. Otherwise, evaluation was carried out under conditions same as the test condition 1.

A result of the performance evaluation test is shown in Table 1 below.

**Table 1**

| | Catalyst | Binder amount (solid content amount ratio) | Pretreatment condition 1 (without oxidative degradation) | | Pretreatment condition 2 (with oxidative degradation) | |
|---|---|---|---|---|---|---|
| | | | Test condition 1 (ultraviolet ray irradiation) | Test condition 2 (dark place) | Test condition 1 (ultraviolet ray irradiation) | Test condition 2 (dark place) |
| Example 1 | Copper (I) oxide and titanium oxide (complexed) | 25 mass % | 92% | 82% | 90% | 79% |
| Example 2 | Copper (I) oxide and titanium oxide (not complexed) | 25 mass % | 83% | 80% | 80% | 57% |
| Example 3 | Copper (I) oxide and tin oxide (complexed) | 25 mass % | 90% | 80% | 74% | 49% |
| Example 4 | Copper (I) oxide | 50 mass % | 85% | 80% | 56% | 50% |
| Example 5 | Copper (I) oxide | 90 mass % | 75% | 73% | 35% | 37% |
| Example 6 | Copper (I) oxide | 96 mass % | 50% | 46% | 14% | 16% |
| Comparative example 1 | - | 100 mass % | 2% | 0% | 0% | 1% |
| Comparative example 2 | Titanium oxide | 50 mass % | 45% | 2% | 47% | 0% |
| Comparative example 3 | Copper (II) oxide | 50 mass % | 32% | 27% | 28% | 21% |

### Filter performance evaluation

Performance evaluation explained below was carried out concerning the samples for evaluation obtained in the example 7 and the comparative example 4 in which the object to be treated was the filter.

### Allergen inactivation test

An allergen (refined dermatophagoides allergen Derf 1 manufactured by ASAHI BREWERIES, LTD.) was added to a buffer solution (a solution obtained by diluting a biochemical buffer solution 20X PBS Tween-20 Buffer manufactured by Takara Bio Inc. by a factor of 20 with hyperpure water) to prepare allergen liquid having concentration of 100 ng/L.

After 1 mL of the allergen liquid was dripped on the sample for evaluation, the sample for evaluation was left untouched for one minute. Subsequently, the allergen liquid was sucked and collected from the sample for evaluation by a method of suction filtration.

Subsequently, 1 mL of deionized water was dripped on the sample for evaluation and then the deionized water was sucked and collected from the sample for evaluation by the method of suction filtration. This operation was repeated three times.

All the liquids collected by the suction from the sample for evaluation were mixed and allergen concentration of obtained mixed liquid was determined by the enzyme-linked immunosorbent assay method (ELISA method). A decreasing rate of the allergen concentration was derived by an expression {A/ (100+A) }×100 (%) from a value (ng/L) obtained by multiplying an allergen concentration measurement value in the mixed liquid with four and the concentration 100 (ng/L) of the allergen liquid before being dripped on the sample for evaluation. The allergen concentration of the mixed liquid is multiplied by four in order to eliminate, in deriving a concentration change, the influence of dilution by cleaning.

A result of the filter performance evaluation test is shown in Table 2 below.

**Table 2**

| | Allergen inactivator carried on filter | Allergen concentration decreasing rate |
|---|---|---|
| Example 7 | Copper (I) oxide | 84% |
| Comparative example 4 | None | 12% |

As shown in the result above, the samples for evaluation in the examples 1 to 7 showed high allergen inactivating action compared with the comparative examples 1 to 4.

## Claims

1. An allergen inactivator containing, as an active component, copper (I) oxide particulates having an average particle diameter of 10 µm or less.

2. The allergen inactivator according to claim 1, further containing a photocatalyst material,
wherein the copper (I) oxide particulates are complexed with the photocatalyst material.

3. The allergen inactivator according to claim 2, wherein conduction band lower end potential of the photocatalyst material is 0.16 V (vs. SHE, pH=0) or less.

4. An allergen inactivator composition comprising: the allergen inactivator according to any one of claims 1 to 3; and a binder component.

5. The allergen inactivator composition according to claim 4, wherein a mass ratio of the binder component to a solid content amount is 95% or less.

6. A coated product comprising:
an object to be treated; and
a coating film that covers the object,
wherein the coating film is formed of the allergen inactivator composition according to claim 4 or 5.

7. An air purification filter comprising:
the allergen inactivator according to any one of claims 1 to 3; and
a filter that carries the allergen inactivator.

8. An allergen inactivating method comprising steps of:
preparing an allergen inactivator containing copper (I) oxide particulates as an active component; and
bringing gas or liquid containing an allergen into contact with surfaces of the copper (I) oxide particulates to degenerate the allergen and extinguish allergen activity.

9. The allergen inactivating method according to claim 8, wherein the allergen inactivator further contains a photocatalyst material, and the copper (I) oxide is complexed with the photocatalyst material.

10. The allergen inactivating method according to claim 9, wherein conduction band lower end potential of the photocatalyst material is 0.16 V (vs. SHE, pH=0) or less.

11. The allergen inactivating method according to any one of claims 8 to 10, further comprising steps of:
depositing the allergen inactivator on a surface of an object to be treated; and
bringing the gas or the liquid containing the allergen into contact with the surfaces of the copper (I) oxide particulates on the surface of the object.

12. The allergen inactivating method according to any one of claims 8 to 10, further comprising steps of:
mixing the allergen inactivator with a binder component to prepare an allergen inactivator composition;
forming a coating filmmade from the allergen inactivator composition on a surface of an object to be treated; and
bringing the gas or the liquid containing the allergen into contact with the surfaces of the copper (I) oxide particulates exposed to a surface of the coating film.

13. The allergen inactivating method according to claim 12, wherein a mass ratio of the binder component to a solid content amount of the allergen inactivator composition is 95% or less.

14. The allergen inactivating method according to any one of claims 8 to 10, further comprising steps of:
preparing a filter as the object;
causing the filter to carry the allergen inactivator; and
causing air to pass through the filter to extinguish allergen activity in the air.

## Amended claims

### Amended claims under Art. 19.1 PCT

**1.** (Original) An allergen inactivator containing, as an active component, copper (I) oxide particulates having an average particle diameter of 10 µm or less.

**2.** (Original) The allergen inactivator according to claim 1, further containing a photocatalyst material,
wherein the copper (I) oxide particulates are complexed with the photocatalyst material.

**3.** (Original) The allergeninactivatoraccording to claim2,wherein conduction band lower end potential of the photocatalyst material is 0.16 V (vs. SHE, pH=0) or less.

**4.** (Original) An allergen inactivator composition comprising: the allergen inactivator according to any one of claims 1 to 3; and a binder component.

**5.** (Original) The allergen inactivator composition according to claim 4, wherein a mass ratio of the binder component to a solid content amount is 95% or less.

**6.** (Original) A coated product comprising:
an object to be treated; and
a coating film that covers the object,
wherein the coating film is formed of the allergen inactivator composition according to claim 4 or 5.

**7.** (Original) An air purification filter comprising:
the allergen inactivator according to any one of claims 1 to 3;
and
a filter that carries the allergen inactivator.

**8.** (Original) An allergen inactivating method comprising steps of:
preparing an allergen inactivator containing copper (I) oxide particulates as an active component; and
bringing gas or liquid containing an allergen into contact with surfaces of the copper (I) oxide particulates to degenerate the allergen and extinguish allergen activity.

**9.** (Original) The allergen inactivating method according to claim 8, wherein the allergen inactivator further contains a photocatalyst material, and the copper (I) oxide is complexed with the photocatalyst material.

**10.** (Original) The allergen inactivating method according to claim 9, wherein conduction band lower end potential of the photocatalyst material is 0.16 V (vs. SHE, pH=0) or less.

**11.** (Original) The allergen inactivating method according to any one of claims 8 to 10 further comprising steps of:
depositing the allergen inactivator on a surface of an object to be treated; and
bringing the gas or the liquid containing the allergen into contact with the surfaces of the copper (I) oxide particulates on the surface of the object.

**12.** (Original) The allergen inactivating method according to any one of claims 8 to 10, further comprising steps of:
mixing the allergen inactivator with a binder component to prepare an allergen inactivator composition;
forming a coating film made from the allergen inactivator composition on a surface of an object to be treated; and
bringing the gas or the liquid containing the allergen into contact with the surfaces of the copper (I) oxide particulates exposed to a surface of the coating film.

**13.** (Original) The allergen inactivating method according to claim 12, wherein a mass ratio of the binder component to a solid content amount of the allergen inactivator composition is 95% or less.

**14.** (Original) The allergen inactivating method according to any one of claims 8 to 10, further comprising steps of:
preparing a filter as the object;
causing the filter to carry the allergen inactivator; and
causing air to pass through the filter to extinguish allergen activity in the air.

**15.** (New) The allergen inactivator according to any one of claims 1 to 3, wherein the allergen inactivator is used in inactivating an allergen excluding a fungus and a virus.

**16.** (New) The allergen inactivator according to any one of claims 1 to 3, wherein the allergen inactivator is used in inactivating an allergen contained in excrement of tick, a corpse of tick or cedar pollen.

**17.** (New) The allergen inactivator composition according to claim 4 or 5, wherein the allergen inactivator composition is used in inactivating an allergen excluding a fungus and a virus.

**18.** (New) The allergen inactivator composition according to claim 4 or 5, wherein the allergen inactivator composition is used in inactivating an allergen contained in excrement of tick, a corpse of tick or cedar pollen.

**19.** (New) The coated product according to claim 6, wherein the coated product is used in inactivating an allergen excluding a fungus and a virus.

**20.** (New) The coated product according to claim 6, wherein the coated product is used in inactivating an allergen contained in excrement of tick, a corpse of tick or cedar pollen.

**21.** (New) The air purification filter according to claim 7, wherein the air purification filter is used in inactivating an allergen excluding a fungus and a virus.

**22.** (New) The air purification filter according to claim 7, wherein the air purification filter is used in inactivating an allergen contained in excrement of tick, a corpse of tick or cedar pollen.

**23.** (New) The allergen inactivating method according to any one of claims 8 to 14, wherein the method is characterized to inactivate an allergen excluding a fungus and a virus.

**24.** (New) The allergen inactivating method according to any one of claims 8 to 14, wherein the method is characterized to inactivate an allergen contained in excrement of tick, a corpse of tick or cedar pollen.
